# EUROPÄISCHE PATENTSCHRIFT

(11) **EP 2 594 312 B1**
(45) Veröffentlichungstag und Bekanntmachung des Hinweises auf die Patenterteilung: **30.08.2017**
(21) Anmeldenummer: 11189360.8
(22) Anmeldetag: 16.11.2011
(51) Int. Cl.: A61M 37/00, A01K 11/00

(54) **Vorrichtung zum Aufstechen eines organischen Gewebes und Antriebsmodul**
Device for punctuation of an organic tissue and drive module
Dispositif de perçage d'un tissu organique et module d'entraînement

(43) Veröffentlichungstag der Anmeldung: 22.05.2013
(73) Patentinhaber: MT.DERM GmbH, 14167 Berlin (DE)
(72) Erfinder: Demjanenko, Dmitrij Dipl. Ing., 12167 Berlin (DE)
(74) Vertreter: Bittner, Thomas L.

(56) Entgegenhaltungen:
- EP-A1- 1 958 659
- DE-A1-102004 009 575
- US-A- 6 065 371
- US-A1- 2005 277 973

## Beschreibung

Die Erfindung betrifft eine Vorrichtung zum Aufstechen eines organischen Gewebes, insbesondere einer menschlichen oder tierischen Haut, und ein Antriebsmodul.

### Hintergrund der Erfindung

Derartige Vorrichtungen werden benutzt, um organisches Gewebe, insbesondere menschliche oder tierische Haut, mit einer oder mehreren Gewebeöffnungen zu versehen. Anschließend kann durch die Gewebeöffnungen gegebenenfalls ein fluider Wirkstoff in das Gewebe eingebracht werden. Beispielsweise dienen derartige Stechvorrichtungen zum Eintrag eines Farbstoffes in Verbindung mit dem Tätowieren oder dem Ausbilden von permanentem Make-up. Aber auch das Einbringen anderer Wirkstoffe wie medizinischer oder kosmetischer Substanzen kann in diesem Zusammenhang vorgesehen sein.

Bei bekannten Stechvorrichtungen wird üblicherweise eine Stecheinrichtung, die mit einer oder mehreren Nadeln versehen ist, an einem Gehäuse gehalten und mit Hilfe einer Antriebseinrichtung im Betrieb oszillierend vor und zurück bewegt, so dass ein wiederholtes Aufstechen des organischen Gewebes stattfindet. Die oszillierende Bewegung von Elementen der Antriebseinrichtung und schließlich der Stecheinrichtung führt dazu, dass die als Handgerät ausgebildete Vorrichtung vibriert. Diese Schwingungen muss der Nutzer dann mit seiner Hand, in welcher er die Stechvorrichtung hält, auffangen. Das Vibrieren der Vorrichtung wird vom Nutzer als unangenehm empfunden.

Aus dem Dokument US 6,065,371 A ist ein Doppel-Nadel-Injektionsapparat bekannt, der zwei unabhängig voneinander angetriebene Nadeln aufweist. Jeder Nadel ist ein Motor mit einer Welle zugeordnet. Die Drehbewegungen des jeweiligen Motors werden mittels der Welle auf eine Kopplung übertragen und in oszillierende Auf- und Abbewegungen der jeweiligen Nadel umwandelt. Die Drehrichtungen der Motoren sind einander entgegengesetzt, also einmal im Uhrzeigersinn und einmal im Gegenuhrzeigersinn, um den Drehimpuls, der ansonsten bei einem nur einmotorigen Apparat auftritt, und somit spürbar ist, auszugleichen und auf diese Weise eine Drift des gesamten Apparats zu verhindern. Es ist also eine Vorrichtung zum Aufstechen eines organischen Gewebes mit einem Gehäuse, einer Stecheinrichtung und einer Antriebseinrichtung offenbart. Die Antriebseinrichtung ist konfiguriert, eine oszillierende Antriebskraft zu erzeugen und auf die Stecheinrichtung einzukoppeln, derart, dass die Stecheinrichtung eine oszillierende Bewegung ausführt. Zu der Antriebseinrichtung ist ein Gegenbauteil gebildet, das gegenläufig oszillierend zur Bewegung der Antriebseinrichtung bewegt wird, um Schwingungen zumindest teilweise zu kompensieren.

Aus dem Dokument EP 1 958 659 A1 sind ein Antriebsmodul für eine Vorrichtung zum lokalen Aufstechen einer menschlichen oder einer tierischen Haut und ein Handgerät bekannt. Von einer Antriebseinrichtung wird eine rotierende Antriebsbewegung erzeugt, die mit Hilfe eines an die Antriebseinrichtung gekoppelten Wandlungsmechanismus in eine linearen Antriebsbewegung gewandelt wird, welche dann in eine auf eine die Haut lokal aufstechende Nadeleinrichtung einkoppelbar ist, so dass diese sich vor und zurück bewegt.

Siehe auch US 2005/277973 A1 und DE 10 2004 009575 A1.

### Zusammenfassung der Erfindung

Aufgabe der Erfindung ist es, eine alternative Vorrichtung zum Aufstechen eines organisches Gewebes sowie ein Antriebsmodul für eine solche Vorrichtung anzugeben, die über einen verbesserten Nutzerkomfort verfügen, insbesondere dadurch, dass Schwingungen der Vorrichtung oder von Elementen hiervon gemindert oder ganz unterbunden sind.

Diese Aufgabe wird erfindungsgemäß durch eine Vorrichtung zum Aufstechen eines organischen Gewebes, insbesondere einer menschlichen oder tierischen Haut, nach dem unabhängigen Anspruch 1 gelöst. Weiterhin ist ein Antriebsmodul für eine Vorrichtung zum Aufstechen eines organischen Gewebes, insbesondere einer menschlichen oder tierischen Haut, nach dem unabhängigen Anspruch 11 geschaffen. Vorteilhafte Ausgestaltungen der Erfindungen sind Gegenstand von abhängigen Unteransprüchen.

Mit der Erfindung sind eine Vorrichtung zum Aufstechen eines organischen Gewebes sowie ein Antriebsmodul für eine solche Vorrichtung geschaffen, bei denen in Betrieb Schwingungen oder Vibrationen des Gehäuses und / oder anderer Elemente der Vorrichtung gemindert oder ganz vermieden sind, indem die Antriebseinrichtung mit einen zusätzlichen Bauteil, nämlich einem Gegen- oder Kompensationsbauteil, gebildet ist, welches bei der oszillierenden Antriebsbewegung ebenfalls oszillierend, aber gegenläufig bewegt wird. Auf diese Weise werden Schwingungskräfte oder -impulse, die aufgrund der oszillierenden Antriebsbewegung, die auch als repetierende Bewegung bezeichnet werden kann, durch Gegenkräfte / -impulse kompensiert, sei es ganz oder teilweise, die von dem zusätzlichen Gegenbauteil in der Antriebseinrichtung bereitgestellt sind. Hierdurch ist eine Verminderung der Vibration der Vorrichtung erreicht.

Das Gehäuse der Vorrichtung kann ein- oder mehrteilig ausgeführt sein. Im Fall einer mehrteiligen Ausführung kann vorgesehen sein, die Vorrichtung mit mehreren voneinander trennbaren Gehäuseteilen gebildet ist, die jeweils mit hierin aufgenommenen funktionellen Komponenten ein oder mehrere funktionelle Module bilden können, insbesondere ein Antriebsmodul mit der Antriebseinrichtung und ein hieran lösbar koppelbares Stech- oder Nadelmodul, welches die Stecheinrichtung aufnimmt. Die Module sind vorzugsweise lösbar miteinander verbunden, zum Beispiel mittels einer Steck- und / oder einer Schraubverbindung. In einer Ausgestaltung kann das Stech- oder Nadelmodul als ein Einwegmodul ausgeführt sein, welches zum Beispiel als Verbrauchsartikel in Form eines sterilisierten Moduls für die einmalige Verwendung zur Verfügung gestellt werden kann. Auch können alternativ oder ergänzend andere Funktionsmodule ans Gehäuse angekoppelt werden, zum Beispiel ein Vorrat oder Tank mit einem fluiden Wirkstoff.

Die Stecheinrichtung kann mit einer Einzelnadel, einer Nadelgruppe und / oder einer Nadelplatte gebildet sein, bei der auf einer nach Außen gerichteten Plattenoberfläche mehrere Nadelspitzen angeordnet sind, beispielsweise auch in Form von Mikronadelspitzen. Die Kopplung der Stecheinrichtung an das Antriebsbauteil erfolgt direkt oder vermittelt über ein oder mehrere Kopplungselemente.

Eine bevorzugte Weiterbildung der Erfindung sieht vor, dass die oszillierende Antriebsbewegung eine axiale Antriebsbewegung ist. Hierbei wird das Antriebsbauteil in Längsrichtung des Gehäuses vor und zurück bewegt. In diesem Zusammenhang kann dann vorgesehen sein, dass auch das Gegenbauteil bei der gegenläufigen oszillierenden Bewegung axial verlagert wird.

Bei einer zweckmäßigen Ausgestaltung der Erfindung kann vorgesehen sein, dass das Antriebsbauteil und das Gegen- oder Kompensationsbauteil in einer kinematischen Kette angeordnet sind. Vorzugsweise sind das Antriebsbauteil und das Gegenbauteil über ein oder mehrere dazwischen angeordnete Kettenglieder verbunden. Die kinematische Kette bewirkt eine kinematische Kopplung von Antriebsbauteil und Gegenbauteil. Eine Bewegung des Antriebsbauteils führt dann zwangsläufig auch zu einer Bewegung des Gegenbauteils. Die kinematische Kopplung ist konfiguriert, eine gegenläufige Bewegung von Antriebsbauteil und Gegenbauteil zu bewirken. Die kinematische Kette ist vorzugsweise als eine offene Kette ausgeführt. In einer Ausgestaltung sind Bauteil und Gegenbauteil über eine ungerade Anzahl von gelenkig verbundenen Kettengliedern verbunden, von denen ein mittleres Kettenglied um einen Drehpunkt drehbar gelagert ist.

Eine vorteilhafte Ausführungsform der Erfindung sieht vor, dass die Masse des Antriebsbauteils im Wesentlichen gleich der Masse des Gegenbauteils ist. In dieser Ausgestaltung werden Antriebsbauteil und Gegenbauteil bei der jeweiligen oszillierenden Bewegung vorzugsweise mit gleicher Schwingungsamplitude verlagert. In einer alternativen Ausgestaltung kann vorgesehen sein, dass die Masse des Gegenbauteils größer als die Masse des Antriebsbauteils ist, zum Beispiel zum wenigstens teilweisen Ausgleich der Masse der Stecheinrichtung, die an das Antriebsbauteil koppelt, und / oder anderer an das Antriebsbauteil koppelnder Bauteile, die bei der oszillierenden Bewegung des Antriebsbauteils mitbewegt werden. Hierbei kann dann vorgesehen sein, dass das Antriebsbauteil und das Gegenbauteil eine gegenläufige Bewegung mit unterschiedlicher Schwingungsamplitude ausführen.

Bevorzugt sieht eine Fortbildung der Erfindung vor, dass dem Antriebsbauteil bei der oszillierenden Antriebsbewegung wenigstens hinsichtlich einer Vorwärtsbewegung oder wenigstens hinsichtlich einer Rückwärtsbewegung ein elastisches Federelement zugeordnet ist. Das elastische Federelement kann zum Beispiel als Druck- oder Zugfederelement ausgeführt sein. Das Federelement wandelt die kinetische Energie des zugeordneten oszillierenden Bauteils, also des Antriebsbauteils oder des Gegenbauteils, in eine potentielle Energie um, beispielsweise in eine mechanische Spannung der Druckfeder, welche dann wieder in kinetische Energie des zugeordneten Bauteils umgewandelt werden kann, wobei jedoch gewisse Verluste auftreten.

Bei einer vorteilhaften Ausgestaltung der Erfindung kann vorgesehen sein, dass dem Gegenbauteil bei der zur oszillierenden Antriebsbewegung gegenläufigen Bewegung wenigstens hinsichtlich einer Vorwärtsbewegung oder wenigstens hinsichtlich einer Rückwärtsbewegung ein elastisches Federelement zugeordnet ist. Das elastische Federelement kann beispielsweise als ein Druck- oder ein Zugfederelement ausgebildet sein. Die vorangehend im Zusammenhang mit dem dem Antriebsbauteil zugeordneten Federelement gemachten Erläuterungen gelten entsprechend.

Eine Weiterbildung der Erfindung kann vorsehen, dass das Gegenbauteil als Passivbauteil ausgeführt ist, welches bei der oszillierenden Antriebsbewegung des Antriebsbauteils passiv mitbewegt wird. Bei dieser Ausführungsform wird das Gegenbauteil passiv von dem Antriebsbauteil mitbewegt.

Eine bevorzugte Weiterbildung der Erfindung sieht vor, dass das Gegenbauteil als Aktivbauteil ausgeführt ist, welches bei der oszillierenden Antriebsbewegung des Antriebsbauteils mittels einer dem Gegenbauteil zugeordneten Bautauteilantriebseinrichtung angetrieben und so aktiv mitbewegt wird. Bei dieser Ausführungsform wird das Gegenbauteil selbst angetrieben, zum Beispiel mit Hilfe eines elektromagnetischen Antriebs, wobei eine mechanisch kontaktfreie Antriebseinrichtung bevorzugt ist.

Bei einer zweckmäßigen Ausgestaltung der Erfindung kann vorgesehen sein, dass die Antriebseinrichtung konfiguriert ist das Antriebsbauteil und / oder das Gegenbauteil mechanisch kontaktfrei anzutreiben. Bei dieser Ausgestaltung sind das Antriebsbauteil und / oder das Gegenbauteil zum Beispiel mechanisch kontaktfrei in einem elektromagnetischen Feld geführt.

In Verbindung mit Antriebsmodul für die Vorrichtung zum Aufstechen eines organischen Gewebes gelten die in Verbindung mit den Fortbildungen der Vorrichtung gemachten Ausführungen entsprechend.

Die Antriebseinrichtung ist konfiguriert, eine lineare oszillierende Antriebskraft bereitzustellen, die direkt auf die Stecheinrichtung eingekoppelt werden kann, also frei von einer kinematischen Bewegungswandlung, wie dies zum Beispiel bei Antreibseinrichtungen notwendig ist, die mittels eines Motors eine Drehantriebsbewegung Es ist dann eine Vorrichtung zum Aufstechen eines organischen Gewebes, insbesondere einer menschlichen oder tierischen Haut, mit einem Gehäuse, einer Stecheinrichtung, die in dem Gehäuse aufgenommen ist, und einer linearen direkten. Antriebseinrichtung geschaffen, die in dem Gehäuse gebildet und konfiguriert ist, eine oszillierende, linear wirkende Antriebskraft zu erzeugen, derart, dass ein von der Antriebseinrichtung umfasstes Antriebsbauteil, welches an die Stecheinrichtung funktionell koppelt, in dem Gehäuse eine lineare oszillierende Antriebsbewegung ausführt, die auf die Stecheinrichtung eingekoppelt wird. Die oszillierende, linear wirkende Antriebskraft ist vorzugsweise axial zur Gehäuseachse ausgerichtet. Es ist auch hier die Antriebseinrichtung mit einem dem Antriebsbauteil zugeordneten Gegenbauteil gebildet, welches in dem Gehäuse durch die oszillierende Antriebsbewegung des Antriebsbauteils verursachte Schwingungen wenigstens teilweise kompensierend gebildet ist, indem das Gegenbauteil bei der oszillierenden Antriebsbewegung des Antriebsbauteils in dem Gehäuse oszillierend und gegenläufig zum Antriebsbauteil bewegt wird.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1a)- 1e): eine schematische Darstellung einer als Handgerät ausgeführten Vorrichtung zum Aufstechen eines organischen Gewebes im Querschnitt,
- Fig. 2: eine schematische Darstellung für eine mechanische Kopplung eines Antriebsbauteils und eines Gegenbauteils,
- Fig. 3: eine schematische Darstellung für eine weitere mechanische Kopplung eines Antriebsbauteils und eines Gegenbauteils,
- Fig. 4: eine schematische Darstellung für eine mechanische Kopplung mittels Zahnstangen und Zahnrad eines Antriebsbauteils und eines Gegenbauteils,
- Fig. 5: eine schematische Darstellung für eine mechanische Kopplung eines Bauteils und eines Gegenbauteils mittels Zahnstangen und mehreren Zahnrädern,
- Fig. 6: eine schematische Darstellung für eine Kopplung eines Antriebsbauteils und eines Gegenbauteils mittels Seilführung,
- Fig. 7: eine schematische Darstellung für eine fluidische Kopplung eines Antriebsbauteils und eines Gegenbauteils,
- Fig. 8: eine schematische Darstellung für eine mechanische Kopplung eines Antriebsbauteils und eines Gegenbauteils mittels Gewindeschnecke und
- Fig. 9: eine schematische Darstellung einer Ausführungsform für eine gegenläufige Bewegung eines Antriebsbauteils und eines Gegenbauteils unter Verwendung von Spulen.

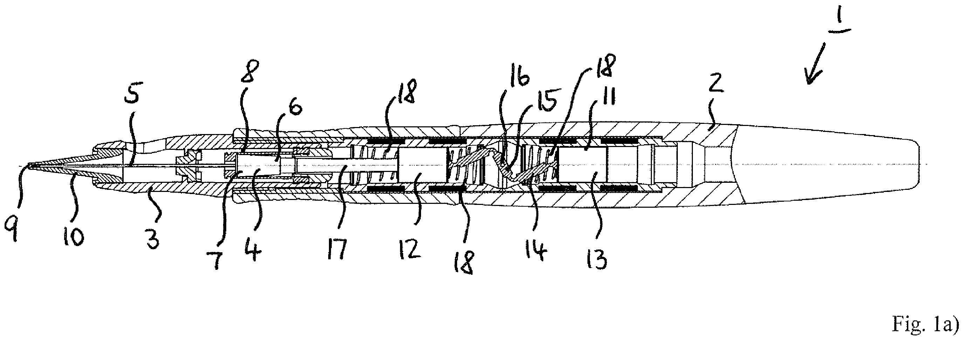

Fig. 1a) bis 1e) zeigen eine als Handgerät ausgeführte Vorrichtung 1 zum Aufstechen eines organischen Gewebes, insbesondere einer menschlichen oder tierischen Haut, im Querschnitt. Die Vorrichtung 1 weist ein Antriebsmodul 2 sowie ein hieran lösbar gekoppeltes Nadelmodul 3 auf. In dem Nadelmodul 3 ist eine Stecheinrichtung 4 angeordnet, die bei der dargestellten Ausführungsform mit einer Nadel 5 gebildet ist. Die Nadel 5 ist rückseitig an einem Nadelträger 6 aufgenommen, der seinerseits mit einem vorderen Abschnitt 7 in einer elastischen Membran 8 gelagert ist. In Betrieb wird die Nadel durch eine Öffnung 9 einer Nadeldüse 10 oszillierend aus- und eingefahren, so dass ein organisches Gewebe aufgestochen werden kann. Hierbei wird die elastische Membran 8, die eine U- oder Topf-Form aufweist, beim Ausfahren der Nadel 5 gestreckt und stellt auf diese Weise eine wahlweise unterstützend wirkende Rückstellkraft für das Zurückbewegen (Wiedereinfahren) des Nadelträgers 6 und der Nadel 8 zur Verfügung. Hierzu ist die elastische Membran 8 im rückseitigen Bereich des Nadelmoduls 3 fixiert.

Im Antriebsmodul 2 ist eine Antriebseinrichtung 11 angeordnet, die bei der dargstellten Ausführungsform mit einem elektrodynamischen Antrieb gebildet ist, der eine lineare Antriebsbewegung direkt zur Verfügung stellt, die dann auf die Stecheinrichtung 4 eingekoppelt wird. Der lineare Antrieb erzeugt im Gegensatz zu einem gewöhnlichen Gleichstrommotor eine lineare repetierende Antriebskraft. Es handelt sich insoweit um einen Direktantrieb. Zum Antrieb der Stecheinrichtung 4 ist keine kinematische Umsetzung der rotierenden Bewegung des Gleichstrommotors in eine lineare Bewegung nötig.

Der Antriebseinrichtung 11 zugeordnet sind ein Antriebsbauteil 12 sowie ein Gegenbauteil 13, welches über Zwischengliedern 14 - drei Zwischenglieder in der gezeigten Ausführungsform - einer kinematischen Kette an das Antriebsbauteil 12 koppelt. Ein mittleres Zwischenglied 15 ist um einen Drehpunkt 16 drehbar gelagert. Antriebsbauteil 12 und Gegenbauteil 13 werden bei der dargestellten Ausführungsform axial oszillierend bewegt. In der gezeigten Ausführungsform koppelt das Antriebsbauteil 12 an einen Stößel 17, welcher seinerseits an den Nadelträger 6 koppelt ist.

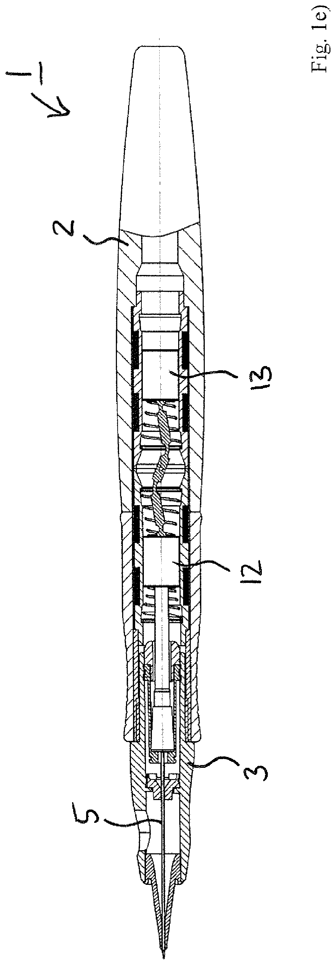

Die Darstellungen in den Fig. 1a) bis 1e) zeigen die kinematische Kette in unterschiedlichen Stellungen, so dass die Nadel 5 in der Darstellung in Fig. 1a) vollständig in das Nadelmodul 3 eingefahren und in der Darstellung in Fig. 1e) vollständig ausgefahren ist.

Die Zwischenglieder 14 bewirken eine kinematische Kopplung des Antriebsbauteils 12, welches seinerseits an den Nadelträger 6 koppelt, und des Gegenbauteils 13, so dass das Gegenbauteil 13 stets axial bewegt wird, wenn das Antriebsbauteil 12 mittels der Antriebseinrichtung 11 angetrieben wird. Die Bewegungen des Antriebsbauteils 12 einerseits und des Gegenbauteils 13 andererseits sind hierbei genau gegenläufig. Dieses führt zu einem wenigstens teilweisen Ausgleich von Schwingungen oder Vibrationen, die bei der oszillierenden Bewegung des Antriebsbauteils 12 erzeugt und auf das Gehäuse der Vorrichtung 1 übertragen werden.

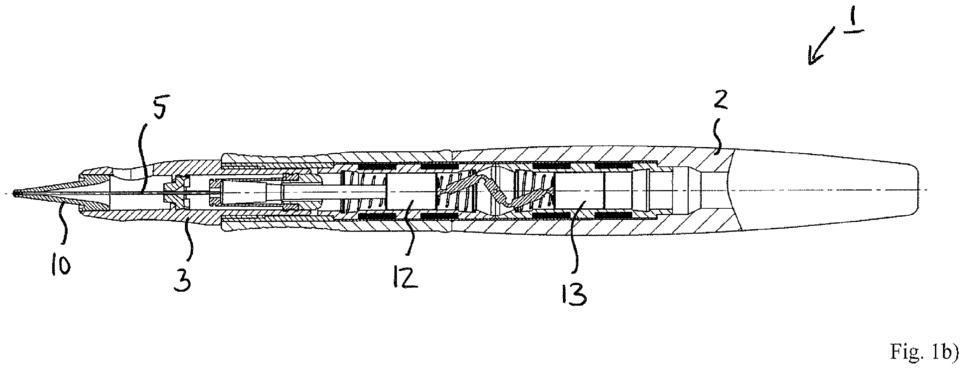

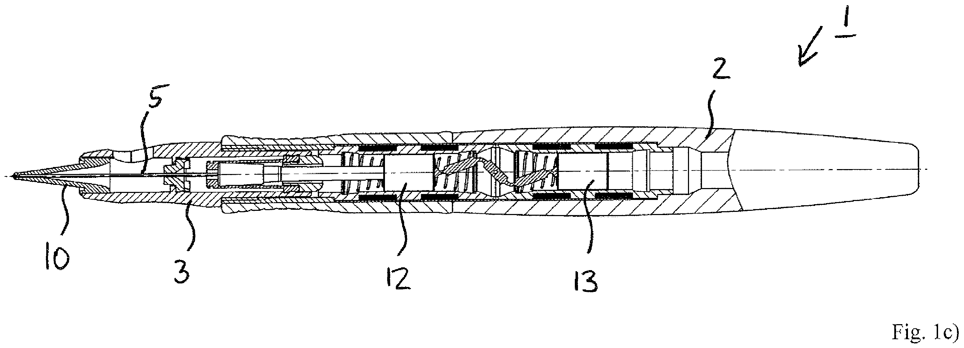

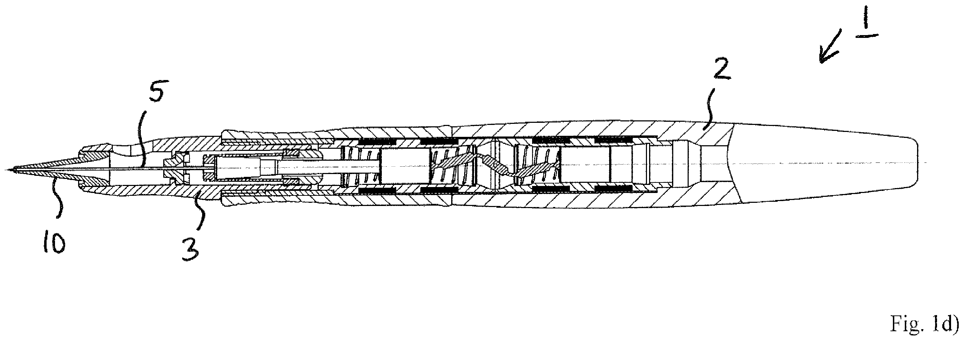

Gemäß der Darstellung in Fig. 1 ist das mittlere Zwischenglied 15 im Drehpunkt 16 drehbar gelagert. Das Antriebsbauteil 12 als auch das Gegenbauteil 13 werden bei der jeweiligen oszillierenden Bewegung gegen elastische Federelemente 18 bewegt. Auf diese Weise ist ein linearer Schwingantrieb gebildet.

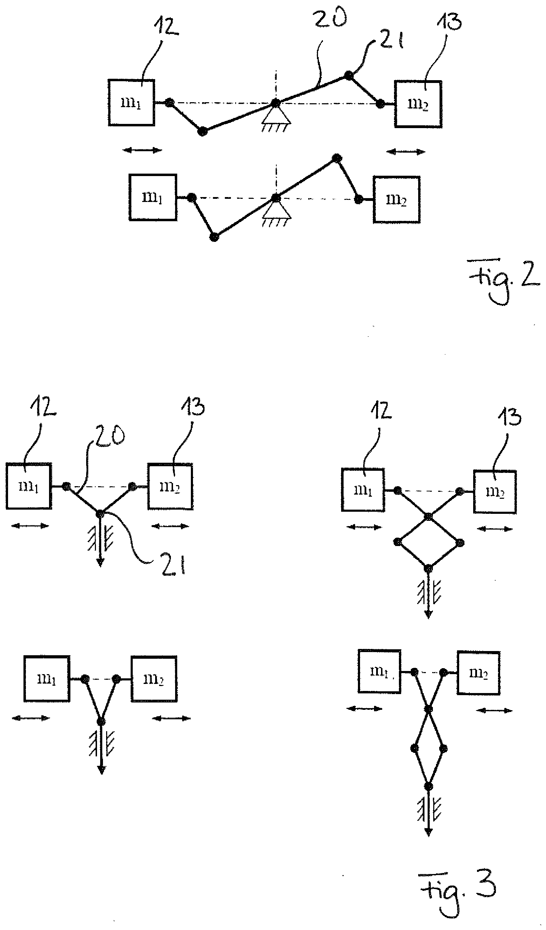

Die Fig. 2 bis 9 zeigen unterschiedliche Ausführungsformen mit dem Antriebsbauteil 12 (m₁) und dem Gegenbauteil 13 (m₂).

Fig. 2 zeigt eine schematische Darstellung für eine mechanische Kopplung des Antriebsbauteils 12 und des Gegenbauteils 13. Die Kopplung zwischen dem Antriebsbauteil 12 und dem Gegenbauteil 13 erfolgt über Hebel 20, die mittels Gelenken 21 miteinander verbunden sind. Die Gelenke 21 können als Gleitlager oder als elastische Verbindungen ausgebildet werden, beispielsweise mittels Silikon oder einem anderen Kunststoff oder mittels Federstahl.

Fig. 3 zeigt eine schematische Darstellung für eine weitere mechanische Kopplung des Antriebsbauteils 12 und des Gegenbauteils 13. Die Kopplung zwischen dem Antriebsbauteil 12 und dem Gegenbauteil 13 erfolgt wieder über Hebel 20, die mittels Gelenken 21 miteinander verbunden sind.

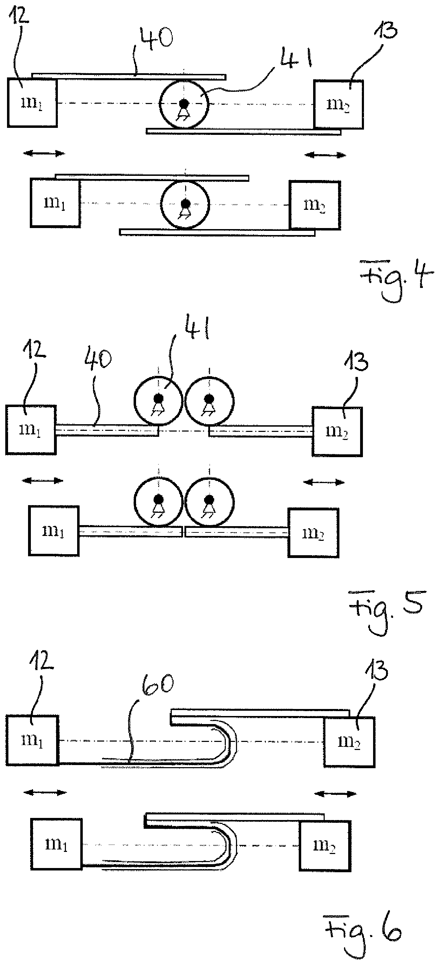

Fig. 4 zeigt eine schematische Darstellung für eine mechanische Kopplung mittels Zahnstangen und Zahnrad des Antriebsbauteils 12 und des Gegenbauteils 13. Die Kopplung der Bauteile erfolgt mittels Zahnstangen 40 und Zahnrad 41.

Fig. 5 zeigt eine schematische Darstellung für eine mechanische Kopplung des Antriebsbauteils 12 und des Gegenbauteils 13 mittels Zahnstangen 40 und mehreren Zahnrädern 41.

Fig. 6 zeigt eine schematische Darstellung für eine Kopplung des Antriebsbauteils 12 und des Gegenbauteils 13 mittels Seilführung 60.

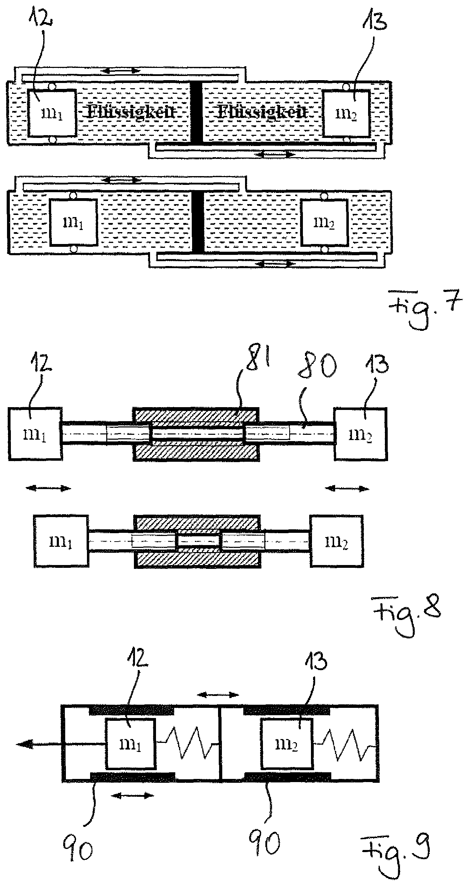

Fig. 7 zeigt eine schematische Darstellung für eine fluidische Kopplung des Antriebsbauteils 12 und des Gegenbauteils 13. Hier ist eine fluidische Kopplung vorgesehen, bei der die sich bewegenden Bauteile ein Fluid verdrängt, welches seinerseits versetzt die Bewegung des jeweils anderen Bauteils bewirkt. Das Fluid ist nur wenig kompressibel oder inkompressibel.

Fig. 8 zeigt eine schematische Darstellung für eine mechanische Kopplung des Antriebsbauteils 12 und des Gegenbauteils 13 mittels Gewindeschnecke 80 mit großer Gewindesteigung, so dass die lineare Bewegung des Antriebsbauteils 12 in einer drehende Bewegung einer Hülse 81 umgesetzt wird, welche ihrerseits wiederum eine lineare Bewegung des Gegenbauteils 13 zur Folge hat.

Fig. 9 zeigt eine schematische Darstellung einer Ausführungsform für eine gegenläufige Bewegung des Antriebsbauteils 12 und des Gegenbauteils 13 unter Verwendung von Spulen 90. Das Antriebsbauteil 12 und das Gegenbauteil 13 sind federnd gelagert und werden jeweils angetrieben mittels der zugeordneten Spule 90. Die gegenläufige Bewegung von Antriebsbauteil 12 und Gegenbauteil 13 wird hier ohne kinematische Kopplung der Bauteile aktiv bewirkt.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Vorrichtung zum Aufstechen eines organischen Gewebes, insbesondere einer menschlichen oder tierischen Haut, mit:
- einem Gehäuse,
- einer Stecheinrichtung (4), die in dem Gehäuse aufgenommen ist, und
- einer linearen direkten Antriebseinrichtung (11), die in dem Gehäuse gebildet und konfiguriert ist, frei von einer kinematischen Bewegungswandlung eine oszillierende, linear wirkende und auf die Stecheinrichtung (4) einkoppelnde Antriebskraft zu erzeugen, derart, dass ein von der linearen direkten Antriebseinrichtung (11) umfasstes Antriebsbauteil (12), welches an die Stecheinrichtung (4) funktionell koppelt, in dem Gehäuse eine lineare oszillierende Antriebsbewegung ausführt, die auf die Stecheinrichtung (4) eingekoppelt wird,
wobei die lineare direkte Antriebseinrichtung (11) mit einem dem Antriebsbauteil (12) zugeordneten Gegenbauteil (13) gebildet ist, welches in dem Gehäuse durch die oszillierende Antriebsbewegung des Antriebsbauteils (12) verursachte Schwingungen wenigstens teilweise kompensierend gebildet ist, indem das Gegenbauteil (13) bei der oszillierenden Antriebsbewegung des Antriebsbauteils (12) in dem Gehäuse linear oszillierend und gegenläufig zum Antriebsbauteil (12) bewegt wird.

2. Vorrichtung nach Anspruch 1, **dadurch gekennzeichnet, dass** die oszillierende Antriebsbewegung eine axiale Antriebsbewegung ist.

3. Vorrichtung nach Anspruch 1 oder 2, **dadurch gekennzeichnet, dass** das Antriebsbauteil (12) und das Gegenbauteil (13) in einer kinematischen Kette angeordnet sind.

4. Vorrichtung nach mindestens einem der vorangehenden Ansprüche, **dadurch gekennzeichnet, dass** die Masse des Antriebsbauteils (12) im Wesentlichen gleich der Masse des Gegenbauteils (13) ist.

5. Vorrichtung nach mindestens einem der vorangehenden Ansprüche, **dadurch gekennzeichnet, dass** dem Antriebsbauteil (12) bei der oszillierenden Antriebsbewegung wenigstens hinsichtlich einer Vorwärtsbewegung oder wenigstens hinsichtlich einer Rückwärtsbewegung ein elastisches Federelement zugeordnet ist.

6. Vorrichtung nach mindestens einem der vorangehenden Ansprüche, **dadurch gekennzeichnet, dass** dem Gegenbauteil (13) bei der zur oszillierenden Antriebsbewegung gegenläufigen Bewegung wenigstens hinsichtlich einer Vorwärtsbewegung oder wenigstens hinsichtlich einer Rückwärtsbewegung ein elastisches Federelement zugeordnet ist.

7. Vorrichtung nach mindestens einem der vorangehenden Ansprüche, dadurch **gekenn- zeichnet,** dass das Gegenbauteil (13) als Passivbauteil ausgeführt ist, welches bei der oszillierenden Antriebsbewegung des Antriebsbauteils (12) passiv mitbewegt wird.

8. Vorrichtung nach mindestens einem der Ansprüche 1 bis 6, **dadurch gekennzeichnet, dass** das Gegenbauteil (13) als Aktivbauteil ausgeführt ist, welches bei der oszillierenden Antriebsbewegung des Antriebsbauteils (12) mittels einer dem Gegenbauteil (13) zugeordneten Bautauteilantriebseinrichtung angetrieben und so aktiv mitbewegt wird.

9. Vorrichtung nach mindestens einem der vorangehenden Ansprüche, **dadurch gekennzeichnet, dass** die lineare direkte Antriebseinrichtung (11) mit einem Schwingantrieb aus der folgenden Gruppe gebildet ist: Antrieb mit Tauchanker, Antrieb mit Zuganker, Parallel-Schwingmotor, Lorentzkraft getriebener Linearantrieb mit beweglicher Spule (Tauchspule) oder beweglichem Läufer (Permanentmagnet), Torsionsschwingantrieb, Piezoantrieb, Ultraschall-Antrieb und pneumatischer Schwingantrieb.

10. Vorrichtung nach mindestens einem der vorangehenden Ansprüche, dadurch **gekenn- zeichnet,** dass die lineare direkte Antriebseinrichtung (11) konfiguriert ist das Antriebsbauteil (12) und / oder das Gegenbauteil (13) mechanisch kontaktfrei anzutreiben.

11. Antriebsmodul (2) für eine Vorrichtung zum Aufstechen eines organischen Gewebes, insbesondere einer menschlichen oder tierischen Haut, mit:
- einem Modulgehäuse und
- einer linearen direkten Antriebseinrichtung (11), die in dem Modulgehäuse gebildet und konfiguriert ist, an eine Stecheinrichtung (4) eines Nadelmoduls (3) zu koppeln und frei von einer kinematischen Bewegungswandlung eine oszillierende, linear wirkende und auf die Stecheinrichtung (4) einkoppelnde Antriebskraft zu erzeugen, derart, dass ein von der linearen direkten Antriebseinrichtung (11) umfasstes Antriebsbauteil (12), welches an die Stecheinrichtung (4) funktionell koppelbar ist, in dem Modulgehäuse eine lineare oszillierende Antriebsbewegung ausführt, die auf die Stecheinrichtung (4) einkoppelbar ist,
wobei die lineare direkte Antriebseinrichtung (11) mit einem dem Antriebsbauteil (12) zugeordneten Gegenbauteil (13) gebildet ist, welches in dem Modulgehäuse durch die oszillierende Antriebsbewegung des Antriebsbauteils (12) verursachte Schwingungen wenigstens teilweise kompensierend gebildet ist, indem das Gegenbauteil (13) bei der oszillierenden Antriebsbewegung des Antriebsbauteils (12) in dem Modulgehäuse linear oszillierend und gegenläufig zum Antriebsbauteil (12) bewegt wird.

## Claims

1. A device for piercing an organic tissue, in particular human or animal skin, comprising:
- a housing,
- a piercing unit (4), which is accommodated in the housing, and
- a linear direct actuating device (11), which is arranged in the housing and which is configured to generate, free of a kinematic movement conversion, an oscillating, linearly acting actuating force which is coupled to the piercing unit (4) such that an actuating component (12) which is surrounded by the linear direct actuating device (11) and which is functionally coupled to the piercing unit (4) performs a linear oscillating actuating movement in the housing which is coupled to the piercing unit (4),
wherein the linear direct actuating device (11) is formed with a counter component (13) which is assigned to the actuating component (12) and in the housing at least partially compensates for the vibrations caused by the oscillating actuating movement of the actuating component (12) in that during the oscillating actuating movement of the actuating component (12) the counter component (13) is moved in an oscillating manner in the housing and contrary to the direction of the actuating component (12).

2. The device in accordance with claim 1 **characterised in that** the oscillating actuating movement is an axial actuating movement.

3. The device in accordance with claim 1 or 2 **characterised in that** the actuating component (12) and the counter component (13) are arranged in a kinematic chain.

4. The device in accordance with at least one of the preceding claims **characterised in that** the mass of the actuating component (12) is essentially equal to the mass of the counter component (13).

5. The device in accordance with at least one of the preceding claims **characterised in that** a spring element is assigned to the actuating component (12) during the oscillating actuating movement at least in terms of a forward movement or at least in terms of a backwards movement.

6. The device in accordance with at least one of the preceding claims **characterised in that** a spring element is assigned to the counter component (13) during the oscillating actuating movement at least in terms of a forward movement or at least in terms of a backwards movement.

7. The device in accordance with at least one of the preceding claims **characterised in that** the counter component (13) is designed as a passive component, which is passively moved during the oscillating actuating movement of the actuating component (12).

8. The device in accordance with at least one of the claims 1 to 6 **characterised in that** the counter component (13) is designed as an active component which during the oscillating actuating movement of the actuating component (12) is actuated by a component actuating device assigned to the counter component (13) and is thus actively moved.

9. The device in accordance with at least one of the preceding claims **characterised in that** the actuating device is designed with a vibration drive from the following group: actuator with plunger anchor, actuator with tension anchor, parallel vibration motor, Lorentz force-driven linear actuator with movable coil (plunger coil) or movable rotors (permanent magnet), torsion vibration actuator, Piezo actuator, ultrasonic actuator and pneumatic vibration actuator.

10. The device in accordance with at least one of the preceding claims **characterised in that** the actuating device (11) is configured to actuate the actuating component (12) and/or the counter component (13) in a mechanically contact-free manner.

11. An actuation module (2) for a device for piercing an organic tissue, in particular human or animal skin, comprising:
- a module housing and
- a linear direct actuating device (11), which is arranged in the module housing and which is configured to couple to a piercing unit (4) of a needle module (3) and to generate, free of a kinematic movement conversion, an oscillating, linearly acting actuating force which is coupled to the piercing unit (4) such that an actuating component (12) which is surrounded by the linear direct actuating device (11) and which is functionally coupled to the piercing unit (4) performs a linear oscillating actuating movement in the module housing which is coupled to the piercing unit (4),
wherein the linear direct actuating device (11) is formed with a counter component (13) which is assigned to the actuating component (12) and in the module housing at least partially compensates for the vibrations caused by the oscillating actuating movement of the actuating component (12) in that during the oscillating actuating movement of the actuating component (12) the counter component (13) is moved in an oscillating manner in the module housing and contrary to the direction of the actuating component (12)

## Revendications

1. Dispositif pour percer un tissu organique, en particulier une peau humaine ou animale, avec :
- un boîtier,
- un dispositif de piqûre (4) réceptionné dans le boîtier, et
- un dispositif d'entraînement direct linéaire (11) qui est formé dans le boîtier et est configuré, en étant libre d'une transformation de mouvement cinématique, pour générer une force d'entraînement oscillante, s'exerçant linéairement et se transmettant au dispositif de piqûre (4) de sorte qu'un élément d'entraînement (12) compris par le dispositif d'entraînement direct linéaire (11), lequel s'accouple fonctionnellement au dispositif de piqûre (4), exécute un mouvement d'entraînement oscillant linéaire dans le boîtier lequel est transmis au dispositif de piqûre (4),
dans lequel le dispositif d'entraînement direct linéaire (11) est formé avec un contre-élément (13) associé à l'élément d'entraînement (12), lequel est réalisé de manière à compenser au moins partiellement des oscillations occasionnées dans le boîtier par le mouvement d'entraînement oscillant de l'élément d'entraînement (12) en ce que, lors du mouvement d'entraînement oscillant de l'élément d'entraînement (12) dans le boîtier, le contre-élément (13) est déplacé de manière à osciller linéairement et en sens contraire à l'élément d'entraînement (12).

2. Dispositif selon la revendication 1, **caractérisé en ce que** le mouvement d'entraînement oscillant est un mouvement d'entraînement axial.

3. Dispositif selon la revendication 1 ou 2, **caractérisé en ce que** l'élément d'entraînement (12) et le contre-élément (13) sont disposés dans une chaîne cinématique.

4. Dispositif selon l'une au moins des revendications précédentes, **caractérisé en ce que** la masse de l'élément d'entraînement (12) est sensiblement égale à la masse du contre-élément (13).

5. Dispositif selon l'une au moins des revendications précédentes, **caractérisé en ce que** l'on associe à l'élément d'entraînement (12), lors du mouvement d'entraînement oscillant, au moins concernant un mouvement vers l'avant ou au moins concernant un mouvement vers l'arrière, un élément de ressort élastique.

6. Dispositif selon l'une au moins des revendications précédentes, **caractérisé en ce que** l'on associe au contre-élément (13), lors du mouvement en sens contraire au mouvement d'entraînement oscillant, au moins concernant un mouvement vers l'avant ou au moins concernant un mouvement vers l'arrière, un élément de ressort élastique.

7. Dispositif selon l'une au moins des revendications précédentes, **caractérisé en ce que** le contre-élément (13) est réalisé en tant qu'élément passif lequel, lors du mouvement d'entraînement oscillant de l'élément d'entraînement (12), est déplacé passivement de façon conjointe.

8. Dispositif selon l'une au moins des revendications 1 à 6, **caractérisé en ce que** le contre-élément (13) est réalisé en tant qu'élément actif lequel, lors du mouvement d'entraînement oscillant de l'élément d'entraînement (12), est entraîné au moyen d'un dispositif d'entraînement d'élément associé au contre-élément (13) et est ainsi déplacé activement de façon conjointe.

9. Dispositif selon l'une au moins des revendications précédentes, **caractérisé en ce que** le dispositif d'entraînement direct linéaire (11) est formé avec un entraînement oscillant du groupe suivant : un entraînement avec un noyau plongeur, un entraînement avec un tirant d'ancrage, un moteur oscillant parallèle, un entraînement linéaire propulsé par force de Lorentz avec une bobine mobile (bobine plongeante) ou un induit mobile (aimant permanent), un entraînement oscillant de torsion, un entraînement piézoélectrique, un entraînement ultrasonore et un entraînement oscillant pneumatique.

10. Dispositif selon l'une au moins des revendications précédentes, **caractérisé en ce que** le dispositif d'entraînement direct linéaire (11) est configuré pour entraîner mécaniquement sans contact l'élément d'entraînement (12) et/ou le contre-élément (13).

11. Module d'entraînement (2) pour un dispositif pour percer un tissu organique, en particulier une peau humaine ou animale, avec :
- un boîtier de module et
- un dispositif d'entraînement direct linéaire (11) qui est formé et configuré dans le boîtier de module pour s'accoupler à un dispositif de piqûre (4) d'un module d'aiguille (3) et pour générer, de manière libre d'une transformation de mouvement cinématique, une force d'entraînement oscillante s'exerçant linéairement et se transmettant au dispositif de piqûre (4) de sorte qu'un élément d'entraînement (12) compris par le dispositif d'entraînement direct linéaire (11), lequel peut être accouplé fonctionnellement au dispositif de piqûre (4), exécute un mouvement d'entraînement oscillant linéaire dans le boîtier de module, lequel peut être transmis au dispositif de piqûre (4),
dans lequel le dispositif d'entraînement direct linéaire (11) est formé avec un contre-élément (13) associé à l'élément d'entraînement (12), lequel est réalisé de manière à compenser au moins partiellement des oscillations occasionnées dans le boîtier de module par le mouvement d'entraînement oscillant de l'élément d'entraînement (12) en ce que, lors du mouvement d'entraînement oscillant de l'élément d'entraînement (12) dans le boîtier de module, le contre-élément (13) est déplacé de manière à osciller linéairement et en sens contraire à l'élément d'entraînement (12).
